Europäisches Patentamt

(19) European Patent Office

Office européen des brevets

(11) EP 0 693 472 B1

(12) FASCICULE DE BREVET EUROPEEN

(45) Date de publication et mention
de la délivrance du brevet:
**14.10.1998 Bulletin 1998/42**

(51) Int. Cl.⁶: $C07C\ 53/16$, $C07C\ 51/41$

(21) Numéro de dépôt: 95401600.2

(22) Date de dépôt: 04.07.1995

(54) **Monochloracetate de sodium ayant des propriétés de mise en oeuvre améliorées**

Natriummonochloracetat mit verbesserten Anwendungseigenschaften

Sodium monochloroacetate having ameliorated applicability

(84) Etats contractants désignés:
**DE ES FR GB IT NL SE**

(30) Priorité: **21.07.1994 FR 9409024**

(43) Date de publication de la demande:
**24.01.1996 Bulletin 1996/04**

(73) Titulaire: **ELF ATOCHEM S.A.**
**92800 Puteaux, Hauts-de-Seine (FR)**

(72) Inventeurs:
  • **Correia, Yves**
    **F-04160 Chateau Arnoux (FR)**
  • **Leduc, Philippe**
    **F-04600 Saint-Auban (FR)**
  • **Jourdain, Dominique**
    **F-40370 Rion des Landes (FR)**

(56) Documents cités:
  DE-B- 1 166 761

  • CHEMICAL ABSTRACTS, vol. 91, no. 11, 10
    Septembre 1979, Columbus, Ohio, US; abstract
    no. 91176e, ENGLUND,B. 'DRY SODIUM
    MONOCHLOROACETATE BY NEUTRALIZING
    MONOCHLOROACETIC ACID WITH ALKALI AND
    DRYING.' page 736 ;colonne 1 ; & SV-A-407 797
    (UDDEHOLMS AB) 23 Avril 1979

## Description

La présente invention concerne un monochloracétate de sodium ayant des propriétés de mise en oeuvre améliorées, notamment, une dissolution rapide dans l'eau et un meilleur écoulement.

Le monochloracétate de sodium est un produit industriel important. Il est utilisé notamment comme réactif intermédiaire dans la synthèse de nombreux produits pharmaceutiques et agrochimiques.

Pour le préparer, de nombreux procédés ont été développés qui consistent essentiellement à neutraliser l'acide monochloracétique par une base telle que la soude ou le carbonate de sodium.

Dans le brevet anglais GB 782479, on propose d'effectuer la neutralisation de l'acide monochloracétique fondu au moyen de lessive de soude concentrée (50 à 55 %), de carbonate de sodium en suspension ou en solution concentrée, ou à l'état pulvérulent dans un sécheur par pulvérisation.

Ainsi l'exemple 5 illustre un procédé qui consiste à introduire le carbonate de sodium avec de l'air à 100°C et à pulvériser simultanément de l'acide monochloracétique fondu à 80°C.

Dans les brevets allemands DE 860354 et DE 871890, on mélange par exemple selon l'équation :

$$2ClCH_2COOH + Na_2CO_3 \rightarrow 2ClCH_2COONa + CO_2 + H_2O \qquad (I)$$

un acide monochloracétique fondu ou cristallin avec du carbonate de sodium anhydre selon des quantités stoechiométriques.

Ensuite, ce mélange est introduit, après une étape de broyage à basse température, dans un solvant afin de terminer la réaction ; ou bien encore l'on fait réagir totalement et rapidement vers 70°C, en éliminant simultanément l'eau formée.

Dans le brevet allemand DE 871890, on obtient après un temps de séjour d'environ 30 minutes un monochloracétate de sodium présentant une teneur en eau de 1 % en poids.

D'une façon générale, tous ces procédés qui s'effectuent soit en absence d'humidité, soit avec élimination rapide de l'humidité (séchage par pulvérisation) conduisent à un monochloracétate de sodium pulvérulent, très fin ayant tendance à motter ce qui occasionne un mauvais écoulement dans les transports pneumatiques ou lors du dépotage.

Par ailleurs, un tel produit pulvérulent se solubilise très lentement et ne facilite pas ainsi les traitements ultérieurs. Ainsi, s'agissant de la solubilisation dans l'eau, des temps de dissolution prolongés sont de nature à provoquer une hydrolyse partielle du monochloracétate de sodium avant que soit terminée ladite dissolution entraînant la formation de produits secondaires tels que le glycolate de sodium la présence desquels est redhibitoire pour une utilisation ultérieure du monochloracétate de sodium.

Dans le brevet allemand DE 2 432 567, il est décrit un procédé de préparation de sels de métal alcalin d'acides chloracétiques et, notamment, de monochloracétate de sodium.

Ce procédé consiste à introduire, par exemple, l'acide monochloracétique et l'agent alcalin aux environs de 90°C dans un lit tourbillonnaire formé à partir du sel qui se forme et d'un courant de gaz inerte utilisé comme véhicule tourbillonnaire, et on soutire le monochloracétate de sodium obtenu, présentant de bonnes caractéristiques d'écoulement, hors du réacteur à lit tourbillonnaire au fur et à mesure de sa formation.

Cependant, on ne donne dans ce brevet aucune illustration desdites caractéristiques d'écoulement, ni du temps de dissolution notamment dans l'eau.

Par ailleurs, on peut remarquer que les produits obtenus possèdent une quantité importante de fines particules ; 50 % à 70 % des particules ont un diamètre allant de 0,20 à 0,063 mm, ce qui n'est pas favorable pour une manipulation aisée du produit.

On a maintenant trouvé un monochloracétate de sodium qui ne présente plus les inconvénients précédemment mentionnés, caractérisé en ce qu'il présente :

a/ au moins 50 % en poids de particules ayant des diamètres allant de 0,6 mm à 2 mm,
b/ un temps de dissolution dans l'eau au plus égal à 17 mn,
c/ une masse volumique apparente au moins égale à 800 kg/m$^3$ et, de préférence allant de 820 kg/m$^3$ à 900 kg/m$^3$, déterminée selon la norme ASTM D 501, en exprimant les résultats en kg/m$^3$, et
d/ une coulabilité au moins égale à 5, et, de préférence allant de 6 à 8, déterminée selon la norme RP PO 121 C.

Selon la présente invention, le monochloracétate de sodium présente de 25 % à 90 % en poids de particules ayant des diamètres supérieurs à 1 mm et, de préférence, au moins 50 % en poids de particules ayant des diamètres allant de 1 mm à 2 mm.

Selon la présente invention, le temps de dissolution qui correspond à la dissolution à température ambiante de 15 g de monochloracétate de sodium dans 50 ml d'eau froide agitée va de préférence de 5 mn à 12 mn.

Le monochloracétate de sodium peut être obtenu par tout procédé qui consiste à neutraliser l'acide monochlora-

2

cétique par un agent alcalin tel que la soude ou le carbonate de sodium à sec ou en solution.

L'obtention du monochloracétate de sodium selon l'invention consiste à soumettre la poudre obtenue selon l'un quelconque de ces procédés aux opérations successives de compactage, de granulation, de criblage puis de tamisage.

L'opération de compactage peut être réalisée sur une presse équipée de deux cylindres tournant en sens inverse à une vitesse généralement comprise entre 5 et 20 tr/mn, appliquant une pression sur la poudre qui est généralement introduite au moyen d'une trémie.

La pression appliquée sur la poudre que l'on désigne communément par pression de compactage peut varier dans une large mesure. Elle est généralement comprise entre 20 bars et 120 bars. L'opération de compactage est réalisée de préférence à une température inférieure à la température de décomposition du monochloracétate de sodium qui se situe aux environs de 110°C. On opère à une température comprise entre 0°C et 60°C et, de préférence voisine de 20°C.

Les cylindres peuvent être éventuellement munis de système de circulation d'eau réfrigérée. La poudre compactée se présente sous forme de plaques - ou galettes- qui sont ensuite granulés dans un granulateur muni d'un rotor.

Dans le granulateur, les plaques sont concassées et désagrégées sous forme de particules lesquelles sont ensuite criblées au moyen de une ou plusieurs grilles de sélection qui possèdent des ouvertures au plus égales à 10 mm et, de préférence, allant de 0,6 mm à 2 mm.

Les fines obtenues peuvent être avantageusement recyclées au système de compactage. Le produit criblé est généralement tamisé. Sur la poudre obtenue, on détermine le temps de dissolution dans l'eau et la masse volumique apparente selon la norme ASTM D 501.

La coulabilité de la poudre obtenue ou, son aptitude à l'écoulement est déterminée selon la norme RP PO 121C. Elle va, avantageusement, de 6 à 8.

La poudre de monochloracétate de sodium obtenue selon la présente invention ne s'agglomère pas. Le mottage est en effet faible, voir quasi nul. Elle se dissout rapidement dans l'eau, ce qui est de nature à faciliter son utilisation ultérieure.

En outre, la poudre de monochloracétate de sodium selon la présente invention contient un taux de fines particules très faible, ce qui assure des conditions de mise en oeuvre et de travail meilleures.

A noter également que la poudre présente une teneur en eau très faible, généralement inférieure à 0,6 %.

Les exemples qui suivent illustrent l'invention.

Préparation des Témoins A, B, C et D

Le monochloracétate de sodium est obtenu par neutralisation de l'acide monochloracétique par le carbonate de sodium selon l'équation (I). On opère en phase solide dans un pétrin aux environs de 70°C.

On obtient une poudre sur laquelle on détermine la masse volumique apparente selon la norme ASTM D 501, la vitesse de dissolution dans l'eau et la teneur en eau. On effectue également sur la poudre obtenue les tests d'écoulement, de mottage et une analyse granulométrique.

**- Détermination de la vitesse de dissolution dans l'eau**

On pèse 15 g (± 0,1 g) de poudre de monochloracétate de sodium que l'on introduit dans un bêcher sec de 150 ml (diamètre : 55 mm, hauteur : 95 mm) muni d'un barreau aimanté mis en mouvement par un agitateur magnétique tournant à 250 tr/mn.

On introduit en une seule fois 50 ml d'eau et on note le temps nécessaire à la dissolution totale de la poudre de monochloracétate de sodium.

**- Test d'écoulement ou détermination de l'aptitude à l'écoulement (coulabilité).**

L'aptitude à l'écoulement a été déterminée selon la norme RP PO 121 C, en utilisant la méthode dite "des sabliers".

La méthode consiste à remplir totalement des sabliers en forme d'entonnoir dont les numéros correspondent à des diamètres différents tels que mentionnés ci-après :

| No du sablier | 2 | 3 | 4 | 5 | 6 | 7 | 8 |
|---|---|---|---|---|---|---|---|
| Diamètre* (mm) | 35 | 30 | 25 | 20 | 15 | 10 | 5 |

\* diamètre infèrieur du sablier.

On enlève ensuite le volet obturateur et on note le numéro du plus petit sablier vidé en moins de 2 minutes.

La coulabilité est donc d'autant meilleure que le numéro du sablier est élevé.

**- Test de mottage**

Le test consiste à former une galette compacte sous contrainte à température ambiante puis à mesurer la tenue du mottage de cette galette jusqu'à sa désagrégation par addition d'une certaine quantité d'eau.

Confection de la galette

On place et on aplanit délicatement 100 g de poudre de monochloracétate de sodium dans un cylindre de diamètre égal à 75 mm et de hauteur égale à 200 mm, puis on introduit ensuite un piston dans ledit cylindre.

On applique ensuite une charge de 10 kg sur ledit piston pendant 30 minutes.

Après confection de la galette (par mottage sous pression), on ajoute un certain poids d'eau (dans une bouteille plastique) jusqu'à destruction de la galette.

C'est le poids d'eau en gramme qui est reporté comme valeur de mottage.

Plus le poids d'eau est élevé, plus le mottage est important.

Analyse granulométrique

On empile des tamis ayant les dimensions de mailles suivantes : 2 mm, 1 mm, 0,8 mm et 0,63 mm.

On pèse 20 g de poudre de monochloracétate de sodium que l'on verse sur l'empilement de tamis. Après avoir secoué l'ensemble pendant un temps déterminé, on repèse les différentes populations bloquées sur les mailles.

Les résultats obtenus sur les poudres témoin à ces différents tests et déterminations sont reportés dans les tableaux 1, 2 et 3.

Exemples selon l'invention

Avec les différentes poudres témoin A, B, C et D, on alimente une presse à compacter, type WP 50 N/75 commercialisée par la société Alexander Werke, équipée d'un granulateur.

Cette presse est munie de 2 rouleaux de diamètre égal à 152 mm tournant en sens inverse à une vitesse de 8 tr/mn (ou 16 tr/mn).

Le compactage est effectué par passage de la poudre de monochloracétate de sodium à travers lesdits rouleaux appliquant une pression sur ladite poudre allant de 20 bars à 120 bars.

Les galettes obtenues sont concassées dans le granulateur à rotor puis le produit concassé passe à travers une ou deux grilles de sélection présentant des ouvertures variant de 0,6 mm à 2 mm, sachant que le produit véhicule d'une grille d'ouverture égale à 2 mm vers une grille d'ouverture inférieure.

Les fines sont recyclées dans la trémie de la presse à compacter et les grosses sont désagrégées. La poudre est ensuite tamisée puis conditionnée dans des sacs.

Sur les poudres ainsi obtenues, on détermine la masse volumique apparente selon la norme ASTM D 501, la vitesse de dissolution dans l'eau et la teneur en eau. On effectue également les tests d'écoulement, de mottage et une analyse granulométrique.

Les résultats sont reportés dans les tableaux 1, 2 et 3.

Dans ces tableaux sont également reportés les pressions de compactage et les grilles de sélection.

Le tableau 1 concerne des poudres de monochloracétate de sodium obtenues à partir du témoin A -exemples 1A, 2A, 3A et 4A-.

Le tableau 2 concerne des poudres de monochloracétate de sodium obtenues à partir du témoin B -exemples 5B, 6B, 7B, 8B et 9B-.

Le tableau 3 concerne des poudres de monochloracétate de sodium à partir du témoin C -exemple 10C- et du témoin D -exemples 11D et 12D-.

TABLEAU 1

| | EXEMPLES | | | | |
|---|---|---|---|---|---|
| | **TEMOIN A** | **1A** | **2A** | **3A** | **4A** |
| PRESSION COMPACTAGE (bar) | | 120 | 120 | 60 | 60 |
| GRILLES DE SELECTION (mm) | | 0 - 2 | 0,8 - 2 | 0 - 2 | 0,8 - 2 |
| TEST ECOULEMENT | < 3 | 7 | 7 | 7 | 7 |
| MASSE VOLUMIQUE APPARENTE (kg/m3) | 781 | 871 | 824 | 861 | 804 |
| TEST DE MOTTAGE | 500 g | léger mottage | léger mottage | léger mottage | léger mottage |
| GRANULOMETRIE (% de particules) | | | | | |
| > 2 mm | 0 | 0 | 0 | 0 | 0 |
| 1 à 2 mm | 1,5 | 31 | 88,5 | 36 | 85,5 |
| 0,8 à 1 mm | 2 | 12,5 | 11,5 | 13 | 11 |
| 0,63 à 0,8 mm | 2 | 14,5 | 0 | 10 | 3,5 |
| < 0,63 mm | 94,5 | 42 | 0 | 41 | 0 |
| Vitesse de dissolution (mn) | 40 | 24 | 17 | 15 | 14 |

TABLEAU 2

| | EXEMPLES | | | | | |
|---|---|---|---|---|---|---|
| | **TEMOIN B** | **5B** | **6B** | **7B** | **8B** | **9B** |
| PRESSION COMPACTAGE (bar) | | 20 | 40 | 60 | 90 | 120 |
| TEST ECOULEMENT | <3 | 7 | 7 | 7 | | 7 |
| TEST DE MOTTAGE | 300 | | | 122 | léger mottage | 0 |
| GRANULOMETRIE (% de particules) | | | | | | |
| > 2 mm | | 0 | 0 | 0 | 0 | 0 |
| 1 à 2 mm | D50 | 81 | 80,5 | 78,5 | 72,5 | 81,5 |
| 0,8 à 1 mm | = | 12 | 13 | 13,5 | 16 | 14 |
| 0,63 à 0,8 mm | 138μm | 5,5 | 5 | 6,5 | 9,5 | 4,5 |
| < 0,63 mm | | 1,5 | 1,5 | 1,5 | 2 | 0 |
| Vitesse de dissolution (mn) | 11 | 6 | 6 | 6 | 6 | 5 |

TABLEAU 3

| | EXEMPLES | | | | |
|---|---|---|---|---|---|
| | TEMOIN C | 10 C | TEMOIN D | 11D | 12D |
| PRESSION COMPACTAGE (bar) | | 120 | | 60 | 120 |
| GRILLES DE SELECTION (mm) | | | | | |
| TEST ECOULEMENT | 4 | 7 | 3 | 7 | 7 |
| MASSE VOLUMIQUE APPARENTE (kg/m3) | 787 | 874 | 781 | 845 | 869 |
| TEST DE MOTTAGE | 229 | léger mottage | 253 | léger mottage | léger mottage |
| GRANULOMETRIE (% de particules) | | | | | |
| > 2 mm | 0 | 0,5 | 0 | 0,5 | 0,5 |
| 1 à 2 mm | 0,5 | 87 | 0,5 | 78 | 89 |
| 0,8 à 1 mm | 1 | 11 | 1 | 18,5 | 8,5 |
| 0,63 à 0,8 mm | 2,5 | 1 | 1 | 2 | 1 |
| < 0,63 mm | 96 | 0,5 | 97,5 | 1 | 1 |
| Vitesse de dissolution (mn) | 26 | 12 | 17 | 11 | 11,5 |

**Revendications**

1. Monochloracétate de sodium caractérisé en ce qu'il présente :

   a/ au moins 50 % en poids de particules ayant des diamètres allant de 0,6 mm à 2 mm,
   b/ un temps de dissolution dans l'eau au plus égal à 17 mn,
   c/ une masse volumique apparente au moins égale à 800 kg/m$^3$ déterminée selon la norme ASTM D 501, en exprimant les résultats en kg/m$^3$, et
   d/ une coulabilité au moins égale à 5 déterminée selon la norme RP PO 121 C.

2. Monochloracétate de sodium selon la revendication 1, caractérisé en ce qu'il présente de 25 % à 90 % en poids de particules ayant des diamètres supérieurs à 1 mm.

3. Monochloracétate de sodium selon la revendication 1 ou 2, caractérisé en ce qu'il présente au moins 50 % en poids de particules ayant des diamètres allant de 1 mm à 2 mm.

4. Monochloracétate de sodium selon la revendication 1, caractérisé en ce qu'il présente un temps de dissolution allant de 5 mn à 12 mn.

5. Monochloracétate de sodium selon la revendication 1, caractérisé en ce qu'il présente une masse volumique apparente allant de 820 kg/m$^3$ à 900 kg/m$^3$, déterminée selon la norme ASTM D 501.

6. Monochloracétate de sodium selon la revendication 1, caractérisé en ce qu'il présente une coulabilité allant de 6 à 8, déterminée selon la norme RP PO 121 C.

7. Procédé d'obtention du monochloracétate de sodium des revendications 1 à 6, caractérisé en ce qu'il consiste à soumettre une poudre obtenue par neutralisation de l'acide monochloracétique aux opérations successives de compactage, de granulation, de criblage puis de tamisage.

**Claims**

1. Sodium monochloroacetate, characterized in that it has:

a/ at least 50% by weight of particles having diameters ranging from 0.6 mm to 2 mm,

b/ a dissolution time in water of not more than 17 min,

c/ an apparent density at least equal to 800 kg/m$^3$, determined according to ASTM standard D 501, the results being expressed in kg/m$^3$, and

d/ a flowability at least equal to 5, determined according to standard RP PO 121 C.

2.  Sodium monochloroacetate according to Claim 1, characterized in that it has from 25% to 90% by weight of particles having diameters greater than 1 mm.

3.  Sodium monochloroacetate according to Claim 1 or 2, characterized in that it has at least 50% by weight of particles having diameters ranging from 1 mm to 2 mm.

4.  Sodium monochloroacetate according to Claim 1, characterized in that it has a dissolution time ranging from 5 min to 12 min.

5.  Sodium monochloroacetate according to Claim 1, characterized in that it has an apparent density ranging from 820 kg/m$^3$ to 900 kg/m$^3$, determined according to ASTM standard D 501.

6.  Sodium monochloroacetate according to Claim 1, characterized in that it has a flowability ranging from 6 to 8, determined according to standard RP PO 121 C.

7.  Process for producing the sodium monochloroacetate of Claims 1 to 6, characterized in that it consists in subjecting a powder obtained by neutralization of monochloroacetic acid to successive compacting, granulation, screening and then sieving operations.

**Patentansprüche**

1.  Natriummonochloracetat, dadurch gekennzeichnet, daß es

    a) mindestens 50 Gew.-% Teilchen mit Durchmessern von 0,6 bis 2 mm,

    b) eine Auflösezeit in Wasser von höchstens 17 Minuten,

    c) eine Schüttdichte von mindestens 800 kg/m$^3$, bestimmt nach der Norm ASTM D 501 unter Berechnung der Ergebnisse in kg/m$^3$, und

    d) eine Fließfähigkeit von mindestens 5, bestimmt nach der Norm RP PO 121 C, aufweist.

2.  Natriummonochloracetat nach Anspruch 1, dadurch gekennzeichnet, daß es 25 Gew.-% bis 90 Gew.-% Teilchen mit Durchmessern oberhalb von 1 mm aufweist.

3.  Natriummonochloracetat nach Anspruch 1 oder 2, dadurch gekennzeichnet, daß es mindestens 50 Gew.-% Teilchen mit Durchmessern von 1 mm bis 2 mm aufweist.

4.  Natriummonochloracetat nach Anspruch 1, dadurch gekennzeichnet, daß es eine Auflösezeit von 5 Minuten bis 12 Minuten aufweist.

5.  Natriummonochloracetat nach Anspruch 1, dadurch gekennzeichnet, daß es eine nach der Norm ASTM D 501 bestimmte Schüttdichte von 820 kg/m$^3$ bis 900 kg/m$^3$ aufweist.

6.  Natriummonochloracetat nach Anspruch 1, dadurch gekennzeichnet, daß es eine nach der Norm RP PO 121 C bestimmte Fließfähigkeit von 6 bis 8 aufweist.

7.  Verfahren zur Herstellung von Natriummonochloracetat nach einem der Ansprüche 1 bis 6, dadurch gekennzeichnet, daß man ein durch Neutralisation von Monochloressigsäure erhaltenes Pulver nacheinander verdichtet, granuliert, klassiert und anschließend siebt.